(19) Europäisches Patentamt
European Patent Office
Office européen des brevets

(11) Veröffentlichungsnummer: **0 002 493**
**B1**

(12) # EUROPÄISCHE PATENTSCHRIFT

(21) Anmeldenummer: **78101580.5**

(22) Anmeldetag: **06.12.78**

(51) Int. Cl.³: **C 07 D 251/16,**
C 07 D 251/26,
C 07 D 403/04

(54) **Verfahren zur Herstellung von 4,6-Dihalogen-1,3,5-triazinen**

(30) Priorität: **17.12.77 DE 2756438**

(43) Veröffentlichungstag der Anmeldung:
**27.06.79 Patentblatt 79/13**

(45) Bekanntmachung des Hinweises auf die Patenterteilung:
**23.07.80 Patentblatt 80/15**

(84) Benannte Vertragsstaaten:
**CH DE FR GB**

(56) Entgegenhaltungen:
**Synthesis, 1978, Seiten 40—42**

(73) Patentinhaber: **Bayer AG**
**Zentralbereich Patente, Marken und Lizenzen**
**D - 5090 Leverkusen 1, Bayerwerk (DE)**

(72) Erfinder: **Findeisen, Kurt, Dr.**
**In der Follmühle 10**
**D - 5068 Odenthal (DE)**

**EP 0 002 493 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Verfahren zur Herstellung von 4,6-Dihalogen-1,3,5-triazinen

Gegenstand der Erfindung ist ein Verfahren zur Herstellung von in 2-Stellung substituierten 4,6-Dihalogen-1,3,5-triazinen.

Es ist bekannt, aus S,S'-Dialkyl-dithiobiureten und Säurechloriden 4,6-Bis-alkylmercapto-1,3,5-triazine, die in 2-Stellung substituiert sind, herzustellen und diese in einer nachfolgenden Umsetzung mit elementarem Chlor in 4,6-Dichlor-1,3,5-triazine, die in 2-Stellung substituiert sind, umzuwandeln [Chem. Ber. *100,* 1874—1891 (1967)].

Es wurde ein Verfahren zur Herstellung von 4,6-Dihalogen-1,3,5-triazinen der allgemeinen Formel I

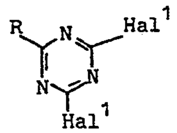

(I),

in der

R einen Aryl- oder Aryloxyrest oder einen 5- oder 6-gliedrigen stickstoffhaltigen aromatischen heterocyclischen Rest bedeutet, der gegebenenfalls einoder mehrfach durch $C_1$—$C_4$-Alkyl, $C_1$—$C_4$-Alkoxy, Halogen, Nitro, Cyano, $C_1$—$C_4$-Carbalkoxy, Aryl oder Aryloxy substituiert sein kann, und

$Hal^1$ ein Halogenatom bedeutet,

gefunden, das dadurch gekennzeichnet ist, daß man Trihalogenmethyl-Verbindungen der allgemeinen Formel II

$$R—C(Hal^2)_3 \qquad (II)$$

in der

R die oben angegebene Bedeutung hat, und

$Hal^2$ ein Halogenatom darstellt, das gleich oder verschieden von $Hal^1$ sein Kann,

mit einem Halogencyan der allgemeinen Formel III

$$Hal^1—CN \qquad (III)$$

in der

$Hal^1$ die oben angegebene Bedeutung hat,

bei Temperaturen von etwa 50 bis 200°C in Gegenwart von Lewis-Säuren und gegebenenfalls in Gegenwart eines inerten Lösungsmittels umsetzt.

Aryl- oder Aryloxyreste (R) leiten sich von einem aromatischen carbocyclischen System, beispeilsweise vom Benzol, Naphthalin oder Anthracen, ab. Bevorzugt werden Aryl- oder Aryloxyreste (R), die sich vom Benzol ableiten. Insbesondere bevorzugt sind der Phenyl- und der Phenoxyrest.

5- oder 6-gliedrige stickstoffhaltige, aromatische, heterocyclische Reste leiten sich beispielsweise vom Pyrrol, Pyrazol, Imidazol, Triazol, Pyridin, Pyrimidin, Pyrazin, Triazin oder Tetrazin ab.

Die Rest R in den allgemeinen Formel I und II können ein- oder mehrfach durch $C_1$—$C_4$-Alkyl, $C_1$—$C_4$-Alkoxy, Halogen, Nitro, Cyano, $C_1$—$C_4$-Carbalkoxy, Aryl oder Aryloxy substituiert sein.

Als $C_1$—$C_4$-Alkyl sei beispielsweise Methyl, Äthyl, Propyl, Isopropyl, Butyl, Isobutyl, bevorzugt Methyl und Äthyl, genannt.

Als $C_1$—$C_4$-Alkoxy sei beispielsweise Methoxy, Äthoxy, Propoxy, Isopropyloxy, Butoxy oder Isobutyloxy, bevorzugt Methoxy und Äthoxy, genannt.

Als Halogen sei beispielswisse Fluor, Chlor, Brom, Jod, bevorzugt Chlor und Brom, genannt.

Als $C_1$—$C_4$-Carbalkoxy seien, beispielsweise die Methyl-, Äthyl-, Propyl-, Isopropyl-, Butyl- oder Isobutyl-Ester von Carbonsäuren genannt, die den Rest R substituieren.

Als Aryl oder Aryloxy seien beispielsweise solche Reste genannt, wie sie für die Definition der Reste R selbst genannt worden sind.

Als $Hal^1$ und $Hal^2$ seien Halogenatome, beispielsweise Fluor, Chlor, Brom oder Jod genannt. Als bevorzugtes Halogenatom $Hal^1$ und $Hal^2$ sei das Chlor genannt.

Als Trihalogenmethyl-Verbindungen der allgemeinen formel II für das erfindungsgemäße Verfahren seien beispielsweise die folgenden genannt:

Benzotrichlorid,
o-, m, p-Chlor-benzotrichlorid,
Dichlor-benzotrichloride,
o-, m-, p-Methoxy-benzotrichlorid,
o-, m-, p-Carbomethoxy-benzotrichlorid,
o-, m-, p-Carbäthoxy-benzotrichlorid,
o-, m-, p-Methyl-benzotrichloride,
o-, p-Phenyl-benzotrichlorid,
Trichlor-methoxybenzol,
o-, m-, p-Chlor-(trichlormethoxy)-benzol,
Dichlor-(trichlormethoxy)-benzole,
o-, m-, p-Nitro-(trichlormethoxy)-benzole,
Chlor-nitro-(trichlormethoxy)-benzole,
o-, m-, p-Cyano-(trichlormethoxy)-benzole,
Benzotribromid,
o-, m-, p-Brom-benzotrichlorid,
o-, m-, p-Brom-benzotribromid,
o-, m-, p-Methoxy-(trichlormethoxy)-benzol,
o-, m-, p-Carbäthoxy-(trichlormethoxy)-benzol,
o-, m-, p-Methyl-(trichlormethoxy)-benzol,
o-, p-Phenyl-(trichlormethyl)-benzol,
o-, m-, p-Methoxy-(tribrommethyl)-benzol,

Als Halogencyane der allgemeinen Formel III seien beispielsweise Fluorcyan, Chlorcyan oder Bromcyan genannt. Bevorzugt für das erfindungsgemäße Verfahren wird das Chlorcyan.

Das erfindungsgemäße Verfahren wird bei Temperaturen von etwa 50 bis etwa 200°C durchgeführt. Bevorzugt wird im Temperaturbereich von etwa 100 bis etwa 150°C gearbeitet.

Das erfindungsgemäße Verfahren wird in Gegenwart von Lewis-Säuren durchgeführt. Als solche kommen die Lewis-Säuren in Betracht,

die für Friedel-Crafts-Reaktionen eingesetzt werden können (vgl. Olah, Friedel-Crafts and Related Reactions, Vol. 1, p. 201, Intersciens, 1963). Beispielsweise seien genannt: Eisen(II)-chlorid, Eisen(III)-chlorid, Zink-chlorid, Zinn(II)-chlorid, Aluminiumchlorid, Zinkcyanid und Borfluorid. Bevorzugt werden im erfindungsgemäßen Verfahren Aluminiumchlorid und Zinkchlorid eingesetzt. Besonders bevorzugt ist der Einsatz von Aluminiumchlorid.

Die Lewis-Säuren werden in einer Menge von 1 bis 15 Mol-%, bezogen auf die eingesetzte Trihalogenmethyl-Verbindung der allgemeinen Formel II, eingesetzt. Bevorzugt ist der Einsatz einer Menge von 3 bis 8 Mol-% der Lewis-Säuren.

Das erfindungsgemäße Verfahren kann ohne Lösungsmittel oder in Gegenwart eines inerten Lösungsmittels durchgeführt werden. Als inerte Lösungsmittel seien beispielsweise diejenigen genannt, die auch für Friedel-Crafts-Reaktionen eingesetzt werden können. Als bevorzugte Lösungsmittel seien beispielsweise gennant: Chlorbenzol, Dichlorbenzole, Trichlorbenzole und Nitrobenzol.

Selbstverständlich können auch die Reaktionsprodukte des erfindungsgemäßen Verfahrens als Lösungsmittel eingesetzt werden.

Das erfindungsgemäße Verfahren kann drucklos oder unter Druck ausgeführt werden. Bei der Ausführung in einem Druckgefäß, beispielsweise in einem Autoklaven, wird bevorzugt unter dem Eigendruck der Reaktionspartner gearbeitet, der sich unter den Reaktionsbedingungen von selbst einstellt.

Das Molverhältnis der im erfinungsgemäßen Verfahren eingesetzten Trihalogenmethyl-Verbindungen der allgemeinen Formel II und der Halogencyane der allgemeinen Formel III beträgt im allgemeinen 1:3.

Das erfindungsgemäße Verfahren kann anhand der Umsetzung von Benzotrichlorid mit Chlorcyan zu 2-Phenyl-4,6-dichlor-1,3,5-triazin durch die folgende Formelgleichung erläutert werden:

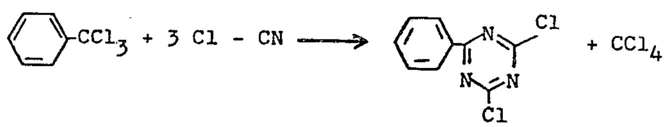

Das erfindungsgemäße Verfahren wird im allgemeinen wie folgt durchgeführt:
Die Trihalogenmethyl-Verbindung der allgemeinen Formel II und die Lewis-Säure werden vorgelegt und auf die Reaktionstemperatur erwärmt. Dann wird unter Einhaltung der Reaktionstemperatur das Halogen-cyan der allgemeinen Formel III zugetropft. Der entstandene Tetrahalogenkohlenstoff wird durch Destillation über eine Kolonne entfernt und das als Rückstand verbleibende erfindungsgemäße Produkt der allgemeinen Formel I durch übliche Aufarbeitung, beispielsweise durch Umkristallisation aus Ligroin, gereinigt.

Die bekannten substituierten 4,6-Dihalogen-1,3,5-triazine sind Zwischenprodukte zur Herstellung von Farbstoffen und Schädlingsbekämpfungsmitteln (siehe z.B. DE—AS 1 026 456).

Während die in 2-Stellung substituierten 4,6-Dichlor-1,3,5-triazine nach dem Stand der Technik [Chem. Ber. *100,* 1874—1891 (1967)] in einer mehrstufigen Synthese erhalten werden können, zeigt das erfindungsgemäße Verfahren einen Weg zu substituierten 4,6-Dihalogen-1,3,5-triazinen in einem Reaktionsschritt unter Verwendung von technisch leicht zugänglichen Verbindungen.

Das erfindungsgemäße Verfahren ist als ausgesprochen überraschend anzusehen, da erwartet werden konnte, daß das eingesetzte Halogencyan der allgemeinen Formel III in Gegenwart von Lewis-Säuren zu Cyanursäurechlorid trimerisieren würde, ohne die Trihalogenmethyl-Verbindung der allgemeinen Formel II in die Reaktion einzubeziehen.

Beispiel 1

140 g 2,4-Dichlor-trichlormethoxybenzol (0,5 Mol) werden in Gegenwart von 4 g Aluminiumchlorid auf 135°C erwärmt und unter Einhaltung dieser Temperatur tropfenweise mit 92, 3 g Chlorcyan (1,5 Mol) versetzt. Nach der Zugabe von ca. 50 g Chlorcyan wird unterbrochen und der entstandene Tetrachlorkohlenstoff abdestilliert, anschließend das restliche Chlorcyan zugetropft. Das dunkel gefärbte Reaktionsprodukt wird fraktioniert destilliert, die festen Reaktionsprodukte umkristallisiert.

Ausbeute: 38 g Tetrachlorkohlenstoff (49% der theoretischen Ausbeute), 70 g 4,6 - Dichlor - 2 - (2',4' - dichlorphenoxy) - 1,3,5-triazin (45% der theoretischen Ausbeutel).

Fp.: 120—122°C

Beispiel 2

97,8 g Benzotrichlorid (0,5 Mol) werden in Gegenwart von 2 g Aluminiumchlorid auf 100°C erwärmt und innerhalb von 20 Minuten mit 92.2 g Chlorcyan (1,5 Mol) tropfenweise versetzt. Die freiwerdende Wärme wird durch gelegentliche Kühlung abgeführt, der entstandene Tetrachlorkohlenstoff durch Destillation entfernt. Nach beendetem Zutropfen wird 30 Minuten bei 130°C nachgerührt, anschließend fraktioniert destilliert.

Ausbeute: 40 g Tetrachlorkohlenstoff (52%

der theoretischen Ausbeute), 69 g 4,6 - Dichlor - 2 - phenyl - 1,3,5 - triazin (61% der theoretischen Ausbeute).

Fp.: 119°C (aus Waschbenzin)

Beispiel 3

115 g 4-Chlor-benzotrichlorid (0,5 Mol) werden in Gegenwart von 4 g Aluminiumchlorid auf 140°C erwärmt und innerhalb von 4 Stunden mit 76 ml Chlorcyan (1,5 Mol) umgesetzt. Man erhält nach der fraktionierten Destillation 46 g Tetrachlorkohlenstoff (≙65% der Theorie) und 91 g 4,6 - Dichloro - 2 - (4′ - chlorphenyl) - 1,3,5 - triazin (70% der theoretischen Ausbeute).

Fp.: 141—143°C (aus Waschbenzin)

Beispiel 4

132 g 2,4-Dichlor-benzotrichlorid (0,5 Mol) werden in Anwesenheit von 5 g Aluminiumchlorid bei 150°C tropfenweise mit 92,2 g Chlorcyan (1,5 Mol) versetzt. Nachdem das Chlorcyan verbraucht ist, werden die niedrigsiedenden Bestandteile abdestilliert, der Rückstand wird aus Waschbenzin umkristallisiert.

Ausbeute: 94 g 4,6 - Dichlor - 2 - (2′,4′ - dichlorphenyl) - 1,3,5 - triazin (64% der theoretischen Ausbeute).

Fp.: 118°C

Beispiel 5

106 g Trichlor-methoxybenzol (0,5 Mol) werden nach Zugabe von 3 g Zinkchlorid bei 120°C tropfenweise innerhalb von 2 Stunden mit 92,2 g Chlorcyan (1,5 Mol) versetzt. Der entstehende Tetrachlorkohlenstoff wird abdestilliert; der Ruckstand aus Waschbenzin umkristallisiert.

Ausbeute: 69 g 4,6 - Dichloro - 2 - phenoxy - 1,3,5 - triazin (57% der theoretischen Ausbeute).

Fp.: 113—114°C

Beispiel 6

97,8 g Benzotrichlorid (0,5 Mol), 2 g Aluminiumchlorid und 92,2 g Chlorcyan (1,5 Mol) werden in einen Autoklav gegeben und 2 Stunden auf 120°C erwärmt. Nach beendeter Umsetzung wird das Reaktionsprodukt fraktionert destilliert; die hochsiedende Fraktion umkristallisiert.

Ausbeute: 77 g 4,6 - Dichlor - 2 - phenyl - 1,3,5 - triazin (68% der theoretischen Ausbeute).

Fp.: 119°C (aus Waschbenzin)

Beispiel 7

In einem Reaktionsgefäß werden 2 g o-Dichlorbenzol und 20 g Aluminiumchlorid auf 140°C erwärmt und innerhalb von 2 Stunden ein Gemisch, bestehend aus 1150 g 4-Chlorbenzotrichlorid (5 Mol) und 992 g Chlorcyan (1,5 Mol), so zugepumpt, daß Chlorcyan immer sofort abreagiert, der entstehende Tetrachlorkohlenstoff wird über eine Kolonne abdestilliert.

Ausbeute: 998 g 2,4 - Dichloro - 2 - (4′ - chlorphenyl) - 1,3,5 - triazin (76% der theoretischen Ausbeute).

Fp.: 142—143°C (aus Waschbenzin)

**Patentansprüche:**

1. Verfahren zur Herstellung von 4,6 - Dihalogen - 1,3,5 - triazinen der allgemeinen Formel I

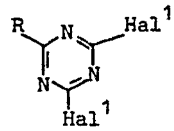

(I),

in der

R einen Aryl- oder Aryloxyrest oder einen 5- oder 6-gliedrigen stickstoffhaltigen aromatischen heterocyclischen Rest bedeutet, der gegebenenfalls einoder mehrfach durch $C_1$—$C_4$-Alkyl, $C_1$—$C_4$-Alkoxy, Halogen, Nitro, Cyano, $C_1$—$C_4$-Carbalkoxy, Aryl oder Aryloxy substituiert sein kann, und

$Hal^1$ ein Halogenatom bedeutet,

dadurch gekennzeichnet, daß man Trihalogenmethyl-Verbindungen der allgemeinen Formel II

$$R—C(Hal^2)_3 \qquad (II)$$

in der

R die oben angegebene Bedeutung hat und

$Hal^2$ ein Halogenatom darstellt, das gleich oder verschieden von $Hal^1$ sein kann,

mit einem Halogencyan der allgemeinen Formel III

$$Hal^1—CN \qquad (III)$$

in der

$Hal^1$ die oben angegebene Bedeutung hat,

bei Temperaturen von etwa 50 bis etwa 200°C in Gegenwart von Lewis-Säuren und gegebenenfalls in Gegenwart eines inerten Lösungsmittels umsetzt.

2. Verfahren nach Anspruch 1, dadurch gekennzeichnet, daß man als Trihalogenmethyl-Verbindungen Trichlormethyl-Verbindungen und als Halogencyan das Chlorcyan einsetzt.

3. Verfahren nach Anspruch 1 und 2, dadurch gekennzeichnet, daß man bei Temperaturen von etwa 100 bis etwa 150°C arbeitet.

**Claims:**

1. Process for the preparation of 4,6-dihalogeno-1,3,5-triazines of the general formula I

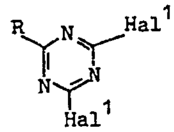

(I),

in which

R denotes an aryl or aryloxy radical or a 5-membered or 6-membered nitrogen-containing aromatic heterocyclic radical and can be optionally monosubstituted or polysubstituted by $C_1$—$C_4$-alkyl, $C_1$—$C_4$-alkoxy, halogen, nitro, cyano, $C_1$—$C_4$-carbalkoxy, aryl or aryloxy and

$Hal^1$ denotes a halogen atom,

characterised in that trihalogenomethyl compounds of the general formula II

$$R—C(Hal^2)_3 \qquad (II)$$

in which

R has the meaning indicated above and

$Hal^2$ represents a halogen atom, which can be identical to or different from $Hal^1$,

are reacted with a cyanogen halide of the general formula III

$$Hal^1—CN \qquad (III)$$

in which

$Hal^1$ has the meaning indicated above,

at temperatures from about 50 to about 200°C in the presence of Lewis acids and optionally in the presence of an inert solvent.

2. Process according to Claim 1, characterised in that trichloromethyl compounds are employed as the trihalogenomethyl compounds and cyanogen chloride is employed as the cyanogen halide.

3. Process according to Claim 1 and 2, characterised in that the reaction is carried out at temperatures from about 100 to about 150°C.

**Revendications**

1. Procédé pour la préparation de 4,6-dihalogéno-1,3,5-triazines de formule générale:

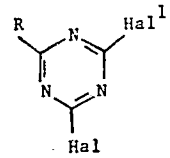

(I)

dans laquelle

R représente un reste aryle ou aryloxy ou un reste aromatique ou hétérocyclique azoté à 5 ou 6 chaînons, qui peut être substitué par un ou plusieurs atomes d'halogène ou groupes alkyle en $C_1$—$C_4$ alcoxy en $C_1$—$C_4$, nitro, cyano, carbalkoxy en $C_1$—$C_4$, aryle ou aryloxy, et

$Hal^1$ est un atome d'halogène,

ledit procédé étant caractérisé en ce que l'on fait réagir des composés trihalogénométhylés de formule générale:

$$R—C(Hal^2)_3 \qquad (II)$$

dans laquelle R est tel que défini ci-dessus, et $Hal^2$ représente un atome d'halogène identique ou différent de $Hal^1$,

avec un halogénure de cyanogène de formule générale:

$$Hal^1—CN \qquad (III)$$

dans laquelle

$Hal^1$ est telque défini ci-dessus

à des températures d'environ 50 à 200°C en présence d'acides de Lewis et éventuellment en présence du'n solvant inerte.

2. Procédé selon la revendication 1, caractérisé in ce que l'on utilise comme composéstrihalogénométhylés des composés trichlorométhylés et comme halogénure de cyanogène le chlorure de cyanogène.

3. Procédé selon la revendication 1 ou 2, caractérisé en ce que l'on opère à des températures d'environ 100 à 150°C.